(19)

Europäisches
Patentamt
European
Patent Office
Office européen
des brevets

(11)  **EP 4 691 514 A1**

(12)  **EUROPEAN PATENT APPLICATION**
published in accordance with Art. 153(4) EPC

(43) Date of publication:
**11.02.2026 Bulletin 2026/07**

(21) Application number: 25788422.1

(22) Date of filing: **06.06.2025**

(51) International Patent Classification (IPC):
*A61M 5/00* (2006.01)

(86) International application number:
**PCT/CN2025/099703**

(87) International publication number:
**WO 2026/001640 (02.01.2026 Gazette 2026/01)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **25.06.2024  CN 202410828438**

(71) Applicant: **Beijing Advanced Medical Technologies, Ltd., Inc.**
**Beijing 102609 (CN)**

(72) Inventors:
• **LIU, Yiwei**
  **Beijing 102629 (CN)**
• **LIU, Qing**
  **Beijing 102629 (CN)**

• **ZHAO, Hugh Qinghong**
  **Beijing 102629 (CN)**
• **NIKANOROV, Alexander**
  **Beijing 102629 (CN)**
• **FENG, Hanqing**
  **Beijing 102629 (CN)**
• **SUN, Hao**
  **Beijing 102629 (CN)**
• **GONG, Xiaofei**
  **Beijing 102629 (CN)**
• **WU, Wen**
  **Beijing 102629 (CN)**
• **LIN, Zhiqi**
  **Beijing 102629 (CN)**

(74) Representative: **Weidner Stern Jeschke**
**Patentanwälte Partnerschaft mbB**
**Wilhelm-Herbst-Straße 5**
**28359 Bremen (DE)**

(54)  **CARBON DIOXIDE ANGIOGRAPHY INJECTOR CONTROL METHOD AND APPARATUS, AND ANGIOGRAPHY INJECTOR**

(57)  The present invention provides a method and a device for controlling a carbon dioxide contrast injector and a contrast injector, which belong to the technical field of carbon dioxide angiography. According to the method for controlling a carbon dioxide contrast injector provided in an embodiment of the present invention, a second length of the spring is determined based on the target injection speed, the initial injection speed, and the first length. On the basis of achieving precise control of the contrast injection volume, the pre-compression degree of the spring can be determined, and then the injection speed and injection volume can be precisely controlled to meet the injection requirements in different situations, thereby improving the accuracy and controllability of $CO_2$ angiography, increasing the injection speed, shortening the injection time, and thus improving the efficiency and speed of the contrast process. The spring in the existing structure is fully utilized, and there is no need to provide an additional pressurizing device, etc., which reduces the modification and cost of the device.

Acquiring a target injection speed and a target injection volume for contrast — 110

Determining a first length of the spring and an initial injection speed corresponding to the first length based on the target injection volume and a sectional area of the filling tube — 120

Determining, when the target injection speed is greater than the initial injection speed, a second length of the spring, based on the target injection speed, the initial injection speed, and the first length — 130

Pre-compressing the spring from the first length to the second length before filling carbon dioxide into the chamber for storing carbon dioxide formed between the end of the filling tube and the piston — 140

Figure 1

## Description

### CROSS REFERENCE TO RELATED APPLICATION

The present invention claims the priority to the Chinese patent application with the filing No. 202410828438.2 filed with the Chinese Patent Office on June 25, 2024, and entitled "METHOD AND DEVICE FOR CONTROLLING A CARBON DIOXIDE CONTRAST INJECTOR AND A CONTRAST INJECTOR", which is incorporated herein by reference in entirety.

### TECHNICAL FIELD

The present invention relates to the technical field of carbon dioxide angiography, and in particular to a method and a device for controlling a carbon dioxide contrast injector and a contrast injector.

### BACKGROUND

Since carbon dioxide naturally exists in the human body, it has good biocompatibility, dissolves in the blood, can be exhaled, and is non-toxic to the liver and kidneys. Therefore, it is often used as a contrast agent for vascular contrast.

In related art, when carbon dioxide is needed for the angiography, it must be injected into the body. The operator typically draws carbon dioxide from a carbon dioxide cylinder into an injector. Then the carbon dioxide is injected into an external injection device communicated to a contrast subject's body, allowing the carbon dioxide to enter the body and achieve vascular contrast.

Due to individual differences in contrast direction and position, a volume and a speed of injected carbon dioxide required during contrast vary. This minimizes discomfort for the contrast subject during injection of carbon dioxide and improves the quality of the final contrast. However, existing carbon dioxide gas injectors simply perform the injection function of carbon dioxide, making it difficult to control the volume of gas used for contrast, let alone the injection speed of the carbon dioxide contrast agent and time of contrast.

### SUMMARY

The present invention provides a method, a device for controlling a carbon dioxide contrast injector and a contrast injector, configured to address an inability to control the injection speed of the contrast in the prior art. The method achieves the effect of pre-compressing a spring to control volume of contrast gas, and injection speed and time of contrast.

The present invention provides a method for controlling a carbon dioxide contrast injector. The contrast injector includes a filling tube for storing and filling carbon dioxide, wherein a piston and a spring for adjusting a capacity of the filling tube are provided within the filling tube, a chamber for storing carbon dioxide is formed between one end of the filling tube, and the piston, a spring is provided at the other end of the filling tube, and the spring pushes the piston to move when it deforms, wherein the method comprises:

acquiring a target injection speed and a target injection volume for the contrast;
determining a first length of the spring and an initial injection speed corresponding to the first length based on the target injection volume and a sectional area of the filling tube, wherein the first length is a length of the spring when a volume of the chamber between the end of the filling tube and the piston is the target injection volume;
determining, when the target injection speed is greater than the initial injection speed, a second length of the spring, based on the target injection speed, the initial injection speed, and the first length; and
pre-compressing the spring from the first length to the second length before filling carbon dioxide into the chamber for storing carbon dioxide formed between the end of the filling tube and the piston, wherein the second length is a length of the spring when a carbon dioxide contrast injection is performed by the filling tube.

According to the method for controlling a carbon dioxide contrast injector provided by the present invention, the determining a first length of the spring and an initial injection speed corresponding to the first length based on the target injection volume and a sectional area of the filling tube includes:

determining a storage position of a carbon dioxide gas within the filling tube based on the target injection volume and the sectional area of the filling tube;
determining the first length of the spring based on the storage position of the carbon dioxide;
obtaining a target pressure difference generated by the spring on the carbon dioxide in the filling tube based on the first length of the spring, an elastic coefficient of the spring, and the sectional area of the filling tube; and
obtaining a gas flow rate of the carbon dioxide based on the target pressure difference and a density of carbon dioxide, and obtaining the initial injection speed based on the gas flow rate of the carbon dioxide.

According to the method for controlling a carbon dioxide contrast injector provided by the present invention, the second length of the spring is determined by the following formula:

$$x_2 = \frac{v_2^2 \cdot x_1}{v_1^2},$$

wherein, $x_1$ is a spring deformation amount at the first length, $x_2$ is a spring deformation amount at the second length, $v_1$ is the initial injection speed, and $v_2$ is the target injection speed.

According to the method for controlling a carbon dioxide contrast injector provided by the present invention, a baffle is provided within the filling tube at an end of the spring close to the piston, a pull rod for adjusting a position of the baffle is fixed to the baffle, and the pull rod extends out of the filling tube from an end of the filling tube away from an injection port; wherein the pre-compressing the spring from the first length to the second length includes:

moving the baffle away from the injection port by the pull rod until the volume of the chamber between the baffle and the filling tube reaches the target injection volume, and then securing the baffle; and compressing an end of the spring away from the injection port until the spring is compressed to the second length.

According to the method for controlling a carbon dioxide contrast injector provided by the present invention, wherein a driving member is provided at an end of the filling tube away from the piston, and an end of the spring away from the injection port is fixedly connected to a driving end of the driving member; and the compressing an end of the spring away from the injection port until the spring is compressed to the second length includes: compressing the end of the spring away from the injection port by driving of the driving member until the spring is compressed to the second length.

The present invention also provides a device for controlling a carbon dioxide contrast injector, including:

an acquisition module configured to acquire a target injection speed and a target injection volume for the contrast;
a first processing module, configured to determine a first length of a spring and an initial injection speed corresponding to the first length based on the target injection volume and a sectional area of a filling tube, wherein the first length is a length of the spring when a volume of a chamber between one end of the filling tube and a piston is the target injection volume;
a second processing module, configured to determine, when the target injection speed is greater than the initial injection speed, a second length of the spring based on the target injection speed, the initial injection speed, and the first length; and
a third processing module, configured to pre-compress the spring from the first length to the second length before filling carbon dioxide into the chamber for storing carbon dioxide formed between the end of the filling tube and the piston, wherein the second length is a length of the spring when a carbon dioxide contrast injection is performed by the filling tube.

The present invention also provides a contrast injector, including a filling tube for storing and filling carbon dioxide, wherein a piston and a spring for adjusting a capacity of the filling tube provided within the filling tube, a chamber for storing carbon dioxide formed between one end of the filling tube and the piston, and the spring is provided at the other end of the filling tube, wherein the spring pushes the piston to move when it deforms; a baffle is provided within the filling tube at an end of the spring close to the piston, a pull rod for adjusting a position of the baffle is fixed to the baffle, and the pull rod extends out of the filling tube from an end of the filling tube away from an injection port; and a driving member provided at an end of the filling tube away from the piston, and an end of the spring away from the injection port is fixedly connected to a driving end of the driving member, wherein prior to a contrast injection, the contrast injector controls the baffle and the driving member by the method for controlling a carbon dioxide contrast injector as mentioned above, so that the spring is compressed to a second length.

The contrast injector provided according to the present invention, wherein the driving member is an adjustment rod for manual adjustment, one end of the adjustment rod is fixedly connected to the end of the spring away from the injection port, and the other end of the adjustment rod extends out the filling tube from an end of the filling tube away from the injection port; alternatively, the driving member is a motor, wherein a driving end of the motor is fixedly connected to the end of the spring away from the injection port.

The present invention also provides an electronic device, including a memory, a processor, and a computer program stored in the memory and executable on the processor, wherein the processor, when executing the program, implements any one of the methods for controlling a carbon dioxide contrast injector as mentioned above.

The present invention also provides a non-transitory computer-readable storage medium, storing a computer program thereon, wherein the computer program, when executed by a processor, implements any one of the methods for controlling a carbon dioxide contrast injector as mentioned above.

The present invention also provides a computer program product including the computer program, wherein the computer program, when executed by a processor, implements any one of the methods for controlling a carbon dioxide contrast injector as mentioned above.

According to the method, a device for controlling a carbon dioxide contrast injector and a contrast injector provided by the present invention, the second length of the spring is determined based on the target injection speed, the initial injection speed, and the first length. On the basis of achieving precise control of the contrast injection volume, the pre-compression degree of the spring can be determined, and then the injection speed and injection volume can be precisely controlled to meet the injection requirements in different situations, thereby improving the accuracy and controllability of $CO_2$ angiography, increasing the injection speed, shortening the injection time, and thus improving the efficiency and speed of the contrast process. The spring in the existing structure is fully utilized, and there is no need to provide an additional pressurizing device, etc., which reduces the modification and cost of the device.

**BRIEF DESCRIPTION OF DRAWINGS**

To more clearly illustrate the technical solutions of the present invention or the prior art, the following briefly introduces the figures required for use in the embodiments or prior art descriptions. Obviously, the figures described below represent some embodiments of the present invention. Those ordinarily skilled in the art can derive other figures based on these figures without inventive effort.

Figure 1 is a first schematic flow chart of a method for controlling a carbon dioxide contrast injector provided in the present invention;
Figure 2 is a second schematic flow chart of the method for controlling a carbon dioxide contrast injector provided in the present invention;
Figure 3 is a first structural schematic diagram of a carbon dioxide contrast injector provided in the present invention;
Figure 4 is a second structural schematic diagram of a carbon dioxide contrast injector provided in the present invention;
Figure 5 is a structural schematic diagram of a device for controlling a carbon dioxide contrast injector provided in the present invention;
Figure 6 is a structural schematic diagram of an electronic device provided in the present invention.

Reference signs:

310: Spring; 320: Piston; 330: Baffle; 340: Pull rod; 350: Filling tube; 360: Chamber; 370: Adjustment rod; 380: Injection port.

**DETAILED DESCRIPTION OF EMBODIMENTS**

To further clarify the objectives, technical solutions, and advantages of the present invention, the technical solutions of the present invention will be described clearly and completely in combination with the drawings in the present invention below. Obviously, the described embodiments represent only a portion, and not all of the embodiments of the present invention. All other embodiments derived by those ordinarily skilled in the art based on the embodiments of the present invention without inventive effort are within the scope of protection of the present invention.

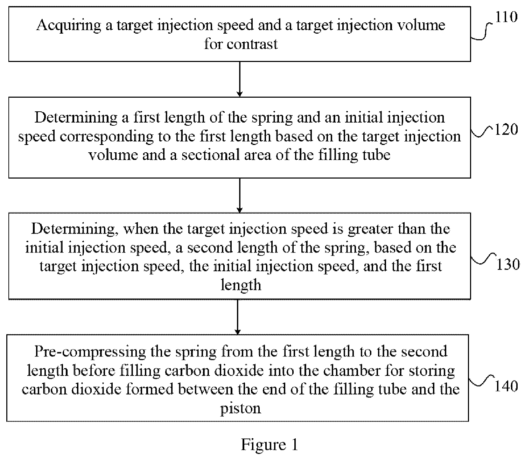

The method, device for controlling a carbon dioxide contrast injector, and contrast injector of the present invention will be described in combination with Figures 1-6 below.

Before describing the method for controlling a carbon dioxide contrast injector of this embodiment in the present invention, the contrast injector of this embodiment will be described.

It will be understood that the contrast injector includes a filling tube for storing and filling carbon dioxide. Before performing angiography on an imaging subject, carbon dioxide gas must be first filled into the filling tube from a gas cylinder, etc., and then injected into the imaging subject through the filling tube.

To control the gas volume of carbon dioxide injected, a piston and a spring for adjusting a capacity of the filling tube is provided in the filling tube. A chamber for storing carbon dioxide is formed between one end of the filling tube and the piston. The spring is provided at the other end of the filling tube, and the piston pushes the piston to move when deformed. By adjusting a compression and extension degree of the spring, the piston is positioned at different positions within the filling tube. The piston at different positions can then be used to control a size of the chamber storing carbon dioxide, thereby controlling the volume of carbon dioxide injected.

As shown in Figure 1, the method for controlling a carbon dioxide contrast injector of the present embodiment mainly includes steps 110, 120, 130, and 140.

Step 110: obtaining a target injection speed and a target injection volume for the contrast.

It is understood that the target injection speed and target injection volume can be determined based on basic information of the imaging subject, such as age, weight, and the specific area to perform contrast.

Contrast operators have accumulated extensive experience in actual operations and can empirically determine the target injection speed and injection volume based on condition and clinical needs of the patient. This method relies on the experience and judgment of medical personnel. Leveraging the intelligent features of modern medical equipment, an automatic parameter confirmation model can analyze the information of imaging subject and determine the target injection speed and injection volume based on the analysis results. This method can improve injection accuracy and controllability.

In other words, the target injection speed and target injection volume can be obtained based on experience or by analyzing the information of the imaging subject through the automatic parameter confirmation model, and are not limited here.

Step 120: determining a first length of the spring and an initial injection speed corresponding to the first length based on the target injection volume and a sectional area of the filling tube.

It is understood that after determining the target injection volume, the position of the piston can be determined based on the target injection volume and the sectional area of the filling tube, thereby ensuring that the chamber between the piston and the filling tube can accommodate the target injection volume of carbon dioxide gas.

After determining the piston position, the position of the spring can be determined, and the first length of the spring can be in turn determined.

In other words, the first length is a length of the spring when the volume of the chamber between one end of the filling tube and the piston is the target injection volume.

It is understood that after determining the required target injection volume, a storage position of carbon dioxide can be calculated through the sectional area and shape of the filling tube, thereby facilitating the determination of the piston position and the first length of the spring.

Based on the target injection volume and the sectional area of the filling tube, the piston position within the filling tube can be calculated. This position ensures that the chamber within the filling tube accommodates the target injection volume of carbon dioxide gas. Once the piston position is determined, the first length of the spring in its current state can be calculated based on the piston position and chamber volume.

By determining these calculations and parameters, it is possible to ensure that the piston and spring are positioned appropriately when injecting $CO_2$ contrast agent, so that the chamber in the filling tube can accurately accommodate the target injection volume of gas, achieving precise control of the injection volume of contrast gas, thereby improving the accuracy and controllability of $CO_2$ angiography.

In some embodiments, the filling tube is communicated with an inlet and outlet tube coaxial with the filling tube at one end of an injection port. A filling valve, which can be a three-way valve, is mounted at an end of the inlet and outlet tube away from the filling tube. An end of the filling valve away from the filling tube is communicated with an insufflation tube. An end of the insufflation tube away from the filling valve is provided with an insufflation assembly. An external injection device communicated with a blood vessel in the body of the patient is also provided on one side of the filling valve.

When carbon dioxide contrast is required, the operator first rotates the filling valve to communicate the insufflation tube with the inlet and outlet tubes. Then, the operator uses the insufflation assembly and the insufflation tube to fill the filling tube with carbon dioxide. After carbon dioxide is filled, the spring is compressed to a first length. At this point, the volume of the chamber for storing carbon dioxide, enclosed between the piston and the filling tube, is the target injection volume. During injection, the filling valve is rotated to communicate the inlet and outlet tubes with the external injection device. The carbon dioxide in the filling tube is then injected into the body of the patient under the pressure of the spring deformation, ultimately enabling the patient to undergo radiological imaging.

In some embodiments, after the first length of the spring is known, the force exerted by the spring on the piston within the chamber and the pressure exerted by the piston on the carbon dioxide can be determined on this basis, and then the flow rate of the carbon dioxide can be obtained, thereby determining the initial injection speed based on the sectional area of an injection pipe.

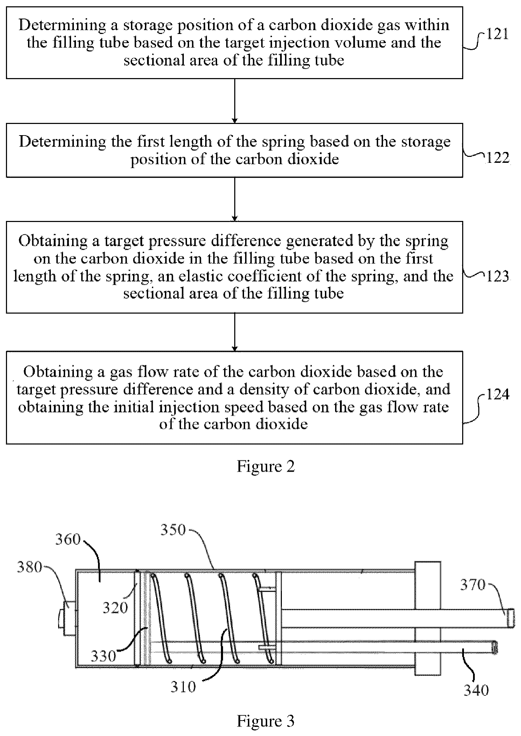

As shown in Figure 2, the determining a first length of the spring and an initial injection speed corresponding to the first length based on the target injection volume and the sectional area of the filling tube includes steps 121, 122, 123, and 124.

Step 121: determining a storage position of a carbon dioxide gas within the filling tube based on the target injection volume and the sectional area of the filling tube.

Step 122: determining the first length of the spring based on the storage position of the carbon dioxide.

Step 123: obtaining a target pressure difference generated by the spring on the carbon dioxide in the filling tube based on the first length of the spring, an elastic coefficient of the spring, and the sectional area of the filling tube.

Step 124: obtaining a gas flow rate of the carbon dioxide based on the target pressure difference and a density of carbon dioxide, and then obtaining the initial injection speed based on the gas flow rate of the carbon dioxide.

It is understood that the target injection volume $V_{tar}$ and the sectional area A of the filling tube are first determined, and the position of the volume of carbon dioxide to be injected in the filling tube (i.e., the position of the piston) can be calculated by dividing these two parameters.

In this case, the position of one end of the spring close to the piston is the position of the piston, so the length of the spring can be determined, that is, the first length of the spring is determined based on the storage position of the carbon dioxide.

It should be noted that according to Hooke's law, the spring force F is proportional to the deformation x, that is, F = kx, where k is an elastic coefficient of the spring. According to Bernoulli's equation and the flow equation, the gas flow rate v and the pressure difference $\Delta P$ satisfy the following fluid dynamics equation:

$$v = \sqrt{\frac{2\Delta P}{\rho}} \quad,$$

where $\rho$ is the gas density, $v$ is the gas flow rate, and $\Delta P$ is the pressure difference between the pressure exerted on carbon dioxide in the filling pipe and the pressure at the injection point.

The force F generated by the spring compression acts on the gas, creating the pressure difference $\Delta P$. The pressure difference can be expressed as:

$$\Delta P = \frac{F}{A} \quad.$$

Substituting the pressure difference formula into the fluid dynamics equation yields:

$$v = \sqrt{\frac{2\Delta P}{\rho}} = \sqrt{\frac{2 \cdot \frac{kx}{A}}{\rho}} \quad;$$

that is,

$$v = \sqrt{\frac{2kx}{A\rho}} \quad.$$

In other words, based on the first length of the spring and the elastic coefficient of the spring, the force F generated by the spring can be obtained. Dividing F by the sectional area A of the filling pipe yields a target pressure difference $\Delta P$ generated by the spring on the $CO_2$ in the filling pipe.

On this basis, the gas flow rate $v$ of carbon dioxide is obtained based on the target pressure difference $\Delta P$ and a density $\rho$ of the carbon dioxide. The initial injection speed in units of volumetric flow rate is then obtained based on the gas flow rate of the carbon dioxide and the sectional area of the injection pipe.

Step 130: determining the second length of the spring, based on the target injection speed, the initial injection speed, and the first length, when the target injection speed is greater than the initial injection speed.

When the target injection speed is greater than the initial injection speed, this indicates that the force generated by the spring at its current deformation degree is too small, which is insufficient to enable carbon dioxide to generate a flow rate enough to meet the requirement of target injection speed.

In this embodiment, the existing structure of the existing carbon dioxide contrast injector is fully utilized, and there is no need to provide an additional pressurizing device, etc. at the injection outlet of the injector. Instead, the injection speed is increased by increasing the deformation degree of the spring and the pressure exerted by the spring on the carbon dioxide.

In this case, a second length of the spring at the target injection speed can be obtained based on the aforementioned relationship formula between gas flow rate and the length of the spring.

That is, the second length of the spring is determined by a following formula:

$$x_2 = \frac{v_2^2 \cdot x_1}{v_1^2} \quad,$$

where $x_1$ is a spring deformation amount at the first length, $x_2$ is a spring deformation amount at the second length, $v_1$ is the initial injection speed, and $v_2$ is the target injection speed.

In this embodiment, the second length of the spring at the target injection speed can be accurately obtained based on the original spring length, the first length, and the corresponding initial injection speed, ensuring that the injection speed meets the requirements.

Step 140: pre-compressing the spring from the first length to the second length before filling carbon dioxide into the chamber for storing carbon dioxide formed between one end of the filling tube and the piston.

Before filling carbon dioxide into the chamber for storing carbon dioxide formed between one end of the filling tube and the piston, the spring can be pre-compressed from the first length to the second length and fixed in a position corresponding to the second length. This ensures that the required initial pressure is maintained in the chamber before filling. During injection, a free end of the spring (i.e., the end connected to the piston) is released, allowing the spring to generate sufficient pressure to expel the carbon dioxide gas, thereby meeting the target injection speed of the carbon dioxide gas.

In this embodiment, the second length is the length of the spring when the carbon dioxide contrast injection is performed by the filling tube.

It is understood that pre-compressing the spring and fixing it at the second length ensures that the required initial pressure is maintained within the chamber before injection, thereby ensuring stability and accuracy during the injection process.

Releasing the free end of the spring allows the spring to generate sufficient pressure to quickly expel the carbon dioxide gas, improving injection speed and efficiency.

By pre-compressing and releasing the spring, the spring compression amount is precisely controllable, thereby accurately controlling the volume of gas released during the injection process.

In this embodiment, this method is relatively simple and intuitive, requiring no complex procedures or device, making it easier for operators to use and master.

According to the method for controlling a carbon dioxide contrast injector provided in embodiments of the present invention, the second length of the spring is determined based on the target injection speed, the initial injection speed, and the first length. On the basis of achieving precise control of the contrast injection volume, the pre-compression degree of the spring can be determined, and then the injection speed and injection volume can be precisely controlled to meet the injection requirements in different situations, thereby improving the accuracy and controllability of $CO_2$ angiography, increasing the injection speed, shortening the injection time, and thus improving the efficiency and speed of the contrast process. The spring in the existing structure is fully utilized, and there is no need to provide an additional pressurizing device, etc., which reduces the modification and cost of the device.

In some embodiments, a baffle is provided within the filling tube at an end of the spring close to the piston. A pull rod for adjusting a position of the baffle is fixed to the baffle. The pull rod extends out of the filling tube from an end of the filling tube away from the injection port. Precompressing the spring from the first length to the second length includes: moving the baffle away from the injection port by the pull rod until the volume of the chamber between the baffle and the filling tube reaches the target injection volume, and then securing the baffle; and compressing an end of the spring away from the injection port until the spring is compressed to the second length.

It should be noted that when the volume of the chamber between the baffle and the filling tube reaches the target injection volume, the baffle is secured, and at this time the spring is compressed to a first length under the action of the baffle. Then, carbon dioxide gas can be injected into the filling tube for storage.

Specifically, carbon dioxide gas can be injected from a carbon dioxide gas source through an injection port into the chamber between the baffle and the filling tube. During the carbon dioxide gas injection process, the piston is squeezed by the gas and gradually moves toward the baffle until the piston and baffle abut against each other. At this point, the chamber is filled with the target injection volume of carbon dioxide gas. On this basis, the end of the spring away from the injection port can be further compressed until the spring is compressed to a second length.

As can be understood, as shown in Figure 3, the end of the spring 310 proximal to the piston 320 is fixedly connected with a baffle 330. The pull rod 340 is fixed to the baffle 330 for adjusting the position of the baffle. The pull rod 340 can extend from the end of the filling tube 350 that secures the spring 310. This will not affect the airtightness of the chamber 360 between the piston 320 and the filling tube 350, and will also facilitate adjustment of the compression degree of the spring 310. This design can achieve the process of pre-compressing the spring 310 from the first length to the second length by moving and fixing the position of the baffle 330.

To secure the baffle to different positions, different scales can be provided on the inner wall of the filling tube to mark different spring lengths. The end of the pull rod extending from the filling tube can be fixed until injection is required, at which point the baffle can be released by unlocking and moving the pull rod. This allows for more precise control of the spring compression level, thereby enabling precise adjustment of the injection speed.

For example, a series of evenly spaced graduations can be uniformly provided on the inner wall of the filling tube, for example, with graduations every 1 mm or 0.5 mm. A locking device can be provided at the end of the pull rod extending from the filling tube to secure the pull rod. For example, a clamp or buckle can be provided to secure the pull rod and baffle. This allows for different injection speeds and is suitable for situations requiring more precise spring length control and injection speed adjustment.

For another example, unequally spaced graduations can be provided based on the changing pattern of spring length, such as densely spaced graduations in areas with less spring compression and fewer graduations in areas with greater compression. This allows for more convenient injection speed adjustment.

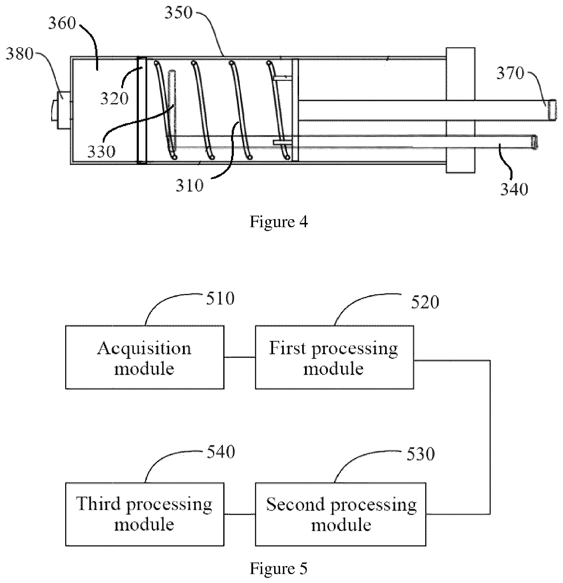

In other embodiments, as shown in Figure 4, the end of the spring 310 close to the piston 320 is fixedly connected to the piston 320, and the baffle 330 is disposed within the spring 310. In this case, the baffle 330 has a small section, allowing the baffle 330 to move within the spring 310, and the movement of the baffle 330 will not affect the movement of the spring 310.

Similarly, a pull rod 340 is fixed to the baffle 330 for adjusting the position of the baffle 330. The pull rod 340 can extend from the end of the filling tube 350 that secures the spring 310. This will not affect the airtightness of the chamber 360 between the piston 320 and the filling tube 350, and will also facilitate adjustment of the compression degree of the spring 310.

In this case, a pull rod is fixed to the baffle for adjusting the position of the baffle. The pull rod extends out the filling tube from the end of the filling tube away from the injection port. Pre-compressing the spring from the first length to the second length includes: moving the baffle away from the injection port by the pull rod until the volume of the chamber between the baffle and the filling tube reaches the target injection volume, and then securing the baffle; and compressing the end of the spring away from the injection port until the spring is compressed to the second length.

It should be noted that when the volume of the chamber between the baffle and the filling tube reaches the target injection volume, the baffle is secured. During the continuous injection of carbon dioxide into the chamber, the piston remains secured by the action of the baffle.

Specifically, carbon dioxide gas can be injected from the carbon dioxide gas source into the chamber between the baffle and the filling pipe through the injection port. During the carbon dioxide gas injection process, the piston is squeezed by the gas and gradually moves toward the baffle until the piston and baffle abut against each other, at which point the chamber is filled with the target injection volume of carbon dioxide gas. Based on this, the end of the spring away from the injection port can be further compressed. The end of the spring fixed to the piston remains fixed in position under the action of the carbon dioxide pressure in the chamber, thereby causing the spring to be compressed until to the second length.

In some embodiments, a driving member is provided at the end of the filling tube away from the piston, and the end of the spring away from the injection port is fixedly connected to a driving end of the driving member. Compressing the end of the spring away from the injection port until the spring is compressed to the second length includes: compressing the end of the spring away from the injection port by driving of the driving member until the spring is compressed to the second length.

It is understood that the driving member is provided at the end of the filling tube fixed to the spring, and the end of the spring fixed to the piston is fixedly connected to the driving end of the driving member.

In some embodiments, the driving member can be an adjustment rod for manual adjustment.

As shown in Figure 3, one end of the adjustment rod 370 is fixedly connected to the end of the spring 310 away from the injection port 380, and the other end of the adjustment rod 370 extends out of the filling tube 350 from the end of the filling tube 350 away from the injection port 380.

The spring can be further compressed by pushing the adjustment rod so that the spring is compressed to a second length. For example, the end of the adjustment rod away from the spring can be provided with a thread, and a wall surface of the filling tube where the adjustment rod passes through the filling tube can be provided with a threaded rod matching the adjustment rod, so that the adjustment rod can also be fixed. That is, only when the adjustment rod is rotated in a direction, the adjusting rod pushes the spring to be compressed. Of course, a fixing locking device can also be provided to fix the movement of the adjustment rod along the axial direction of the filling tube. The fixing method of the adjustment rod is not limited here.

Alternatively, the driving member can be a motor, with a driving end of the motor fixedly connected to the end of the spring away from the injection port.

In this case, a power source needs to be provided in the filling tube to supply power to the driving member. A button can be provided on the outside of the filling tube to adjust the distance the driving member drives the spring, thereby controlling the degree of spring compression.

The control button can be mounted in a suitable position on the outside of the filling tube, such as on the side or top of the filling tube, so that the operator can easily use the buttons. When designing the function of the button, it can be configured as a button for increasing or decreasing the compression degree, or as a button for adjusting the compression degree. The operator can select the appropriate button for adjustment as needed.

The shape and size of the button should be designed to make it easy to operate and identify. For example, a raised button or a button with a clear marking can be used to prevent accidental operations during operation.

In this embodiment, the spring is compressed to a second length by driving of the driving member, thereby realizing control over the pre-compression of the spring. The spring length can be adjusted more conveniently, thereby achieving precise control of the injection speed.

The following describes the device for controlling a carbon dioxide contrast injector provided by the present invention. The device for controlling a carbon dioxide contrast injector described below can be referenced in conjunction with the method for controlling a carbon dioxide contrast injector described above.

As shown in Figure 5, the device for controlling a carbon dioxide contrast injector of this embodiment of the application primarily includes an acquisition module 510, a first processing module 520, a second processing module 530, and a third processing module 540.

The acquisition module 510 is configured to acquire a target injection speed and a target injection volume for contrast.

The first processing module 520 is configured to determine a first length of the spring and an initial injection speed corresponding to the first length based on the target injection volume and the sectional area of the filling tube, wherein the first length is a length of the spring when the volume of the chamber between one end of the filling tube and the piston is the target injection volume.

The second processing module 530 is configured to determine a second length of the spring based on the target injection speed, the initial injection speed, and the first length, when the target injection speed is greater than the initial injection speed.

The third processing module 540 is configured to pre-compress the spring from the first length to the second length before filling carbon dioxide into the chamber for storing carbon dioxide formed between one end of the filling tube and the piston, wherein the second length is the length of the spring when the carbon dioxide contrast injection is performed by the filling tube.

According to the device for controlling a carbon dioxide contrast injector provided in an embodiment of the present invention, the second length of the spring is determined based on the target injection speed, the initial injection speed, and the first length. On the basis of achieving precise control of the contrast injection volume, the pre-compression degree of the spring can be determined, and then the injection speed and injection volume can be precisely controlled to meet the injection requirements in different situations, thereby improving the accuracy and controllability of $CO_2$ angiography, increasing the injection speed, shortening the injection time, and thus improving the efficiency and speed of the contrast process. The spring in the existing structure is fully utilized, and there is no need to provide an additional pressurizing device, etc., which reduces the modification and cost of the device.

The embodiment of the present invention also provides a contrast injector, including a filling tube for storing and filling carbon dioxide. A piston and a spring for adjusting the capacity of the filling tube is provided in the filling tube. A chamber for storing carbon dioxide is formed between one end of the filling tube and the piston. The spring is disposed at the other end of the filling tube, and the piston pushes the piston to move when deformed. A baffle is provided at the end of the spring close to the piston, and a pull rod for adjusting the position of the baffle is fixed to the baffle. The pull rod extends out the filling tube from the end of the filling tube away from the injection port. A driving member is provided at the end of the filling tube away from the piston, and the end of the spring away from the injection port is fixedly connected to a driving end of the driving member. Prior to a contrast injection, the contrast injector controls the baffle and the driving member by the aforementioned method for controlling carbon dioxide contrast injector, so that the spring is compressed to a second length.

In some embodiments, the driving member is an adjustment rod for manual adjustment. One end of the adjustment rod is fixedly connected to the end of the spring away from the injection port, and the other end of the adjustment rod extends out the filling tube from the end of the filling tube away from the injection port. Alternatively, the driving member is a motor, with the driving end of the motor fixedly connected to the end of the spring away from the injection port.

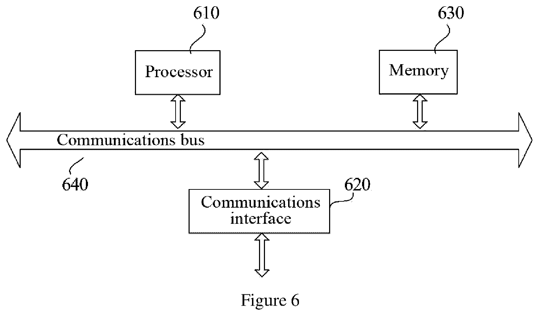

Figure 6 illustrates a schematic diagram of the physical structure of an electronic device. As shown in Figure 6, the electronic device can include: a processor 610, a communications interface 620, a memory 630, and a communications bus 640. The processor 610, the communications interface 620, and the memory 630 communicate with each other via the communications bus 640. The processor 610 can invoke logic instructions in the memory 630 to execute a method for controlling a carbon dioxide contrast injector. The method includes: obtaining a target injection speed and a target injection volume for the contrast; determining a first length of a spring and an initial injection speed corresponding to the first length based on the target injection volume and the sectional area of the filling tube; the first length being a length of the spring when a volume of the chamber between one end of the filling tube and the piston is the target injection volume; determining a second length of the spring based on the target injection speed, the initial injection speed, and the first length, when the target injection speed is greater than the initial injection speed; and pre-compressing the spring from the first length to the second length before filling carbon dioxide into the chamber for storing carbon dioxide formed between one end of the filling tube and the piston; the second length being a length of the spring when a carbon dioxide contrast injection is performed by the filling tube.

In addition, the logic instructions in the aforementioned memory 630 can be implemented as software functional units and, when sold or used as independent products, stored in a computer-readable storage medium. Based on this understanding, the essence of technical solution of the present invention, or the portion that contributes to the prior art, or a portion of the technical solution, can be embodied in the form of a software product. This computer software product, stored in a storage medium, includes several instructions for enabling a computer device (such as a personal computer, server, or network device) to perform all or part of the steps of the methods described in various embodiments of the present invention. The aforementioned storage medium includes various media capable of storing program code, such as USB flash drives, external hard drives, read-only memories (ROMs), random access memories (RAMs), magnetic disks, or optical disks.

In another aspect, the present invention further provides a computer program product. The computer program product includes a computer program that can be stored on a non-transitory computer-readable storage medium. When the computer program is executed by a processor, the computer can execute a method for controlling a carbon dioxide contrast injector provided by each of the above methods. The method includes: obtaining a target injection speed and a target injection volume for the contrast; determining a first length of a spring and an initial injection speed corresponding to the first length based on the target injection volume and the sectional area of the filling tube; the first length being the length of the spring when the volume of the chamber between one end of the filling tube and the piston is the target injection volume; determining a second length of the spring based on the target injection speed, the initial injection speed, and the first length when the target injection speed is greater than the initial injection speed; and pre-compressing the spring from the first length to the second length before filling carbon dioxide into the chamber for storing carbon dioxide formed between one end of the filling tube and the piston; the second length being a length of the spring when a carbon dioxide contrast injection is performed by the filling tube.

In another aspect, the present invention further provides a non-transitory computer-readable storage medium, a computer program is stored on the non-transitory computer-readable storage medium. The computer program, when executed by a processor, implements the method for controlling a carbon dioxide contrast injector provided by the aforementioned methods. The method includes: obtaining a target injection speed and a target injection volume for the contrast; determining a first length of a spring and an initial injection speed corresponding to the first length based on the target injection volume and the sectional area of the filling tube; the first length being the length of the spring when the volume of the chamber between one end of the filling tube and the piston is the target injection volume; determining a second length of the spring based on the target injection speed, the initial injection speed, and the first length when the target injection speed is greater than the initial injection speed; and pre-compressing the spring from the first length to the second length before filling carbon dioxide into the chamber for storing carbon dioxide formed between one end of the filling tube and the piston; the second length being a length of the spring when a carbon dioxide contrast injection is performed by the filling tube.

The device embodiments described above are merely illustrative, wherein the units described as separate components can or cannot be physically separate, and the components shown as units can or cannot be physical units, i.e., they can be located in one position or distributed across multiple network units. Some or all of the modules can be selected based on actual needs to achieve the objectives of the present embodiments. Those ordinarily skilled in the art can understand and implement these embodiments without inventive effort.

Through the above description of the embodiments, those skilled in the art will clearly understand that each embodiment can be implemented using software and a necessary general-purpose hardware platform, or alternatively, hardware. Based on this understanding, the essence of the above technical solution, or the portion that contributes to the prior art, can be embodied in the form of a software product. This software product can be stored in a computer-readable storage medium, such as ROM/RAM, a magnetic disk, or an optical disk, and includes several instructions for enabling a computer device (such as a personal computer, a server, or a network device) to execute the methods described in each embodiment or portions of embodiments.

Finally, it should be noted that the above embodiments are intended only to illustrate the technical solutions of the present invention and are not intended to limit them. Although the present invention has been described in detail with reference to the aforementioned embodiments, those ordinarily skilled in the art should understand that modifications to the technical solutions described in the aforementioned embodiments can be made, or some of the technical features therein can be replaced by equivalents. Such modifications or replacements do not deviate from the essence of the corresponding technical solutions from the spirit and scope of the technical solutions of the various embodiments of the present invention.

**Claims**

1. A method for controlling a carbon dioxide contrast injector, **characterized in that** the contrast injector comprises a filling tube for storing and filling carbon dioxide, wherein a piston and a spring for adjusting a capacity of the filling tube are provided within the filling tube, a chamber for storing carbon dioxide is formed between one end of the filling tube and the piston, the spring is provided at the other end of the filling tube, and the spring pushes the piston to move when it deforms, wherein the method comprises steps of:

   acquiring a target injection speed and a target injection volume for contrast;
   determining a first length of the spring and an initial injection speed corresponding to the first length based on the target injection volume and a sectional area of the filling tube, wherein the first length is a length of the spring when a volume of the chamber between the end of the filling tube and the piston is the target injection volume;
   determining, when the target injection speed is greater than the initial injection speed, a second length of the spring, based on the target injection speed, the initial injection speed, and the first length; and
   pre-compressing the spring from the first length to the second length before filling carbon dioxide into the chamber for storing carbon dioxide formed between the end of the filling tube and the piston, wherein the second length is a length of the spring when a carbon dioxide contrast injection is performed by the filling tube.

2. The method for controlling a carbon dioxide contrast injector according to claim 1, **characterized in that** the step of determining a first length of the spring and an initial injection speed corresponding to the first length based on the target injection volume and a sectional area of the filling tube comprises:

   determining a storage position of a carbon dioxide gas within the filling tube based on the target injection volume and the sectional area of the filling tube;
   determining the first length of the spring based on the storage position of the carbon dioxide;
   obtaining a target pressure difference generated by the spring on the carbon dioxide in the filling tube based on the first length of the spring, an elastic coefficient of the spring, and the sectional area of the filling tube; and
   obtaining a gas flow rate of the carbon dioxide based on the target pressure difference and a density of carbon dioxide, and obtaining the initial injection speed based on the gas flow rate of the carbon dioxide.

3. The method for controlling a carbon dioxide contrast injector according to claim 1, **characterized in that** the second length of the spring is determined by a following formula:

$$x_2 = \frac{v_2^2 \cdot x_1}{v_1^2},$$

wherein, $x_1$ is a spring deformation amount at the first length, $x_2$ is a spring deformation amount at the second length, $v_1$ is the initial injection speed, and $v_2$ is the target injection speed.

4. The method for controlling a carbon dioxide contrast injector according to any one of claims 1-3, **characterized in that** a baffle is provided within the filling tube at an end of the spring close to the piston, a pull rod for adjusting a position of the baffle is fixed to the baffle, and the pull rod extends out of the filling tube from an end of the filling tube away from an injection port, wherein the step of pre-compressing the spring from the first length to the second length comprises steps of:

moving the baffle away from the injection port by the pull rod until the volume of a chamber between the baffle and the filling tube reaches the target injection volume, and then securing the baffle; and
compressing an end of the spring away from the injection port until the spring is compressed to the second length.

5. The method for controlling a carbon dioxide contrast injector according to claim 4, **characterized in that** a driving member is provided at an end of the filling tube away from the piston, and an end of the spring away from the injection port is fixedly connected to a driving end of the driving member; and the step of compressing an end of the spring away from the injection port until the spring is compressed to the second length comprises:
compressing the end of the spring away from the injection port by driving of the driving member, until the spring is compressed to the second length.

6. A device for controlling a carbon dioxide contrast injector, **characterized in that** it comprises:

an acquisition module, configured to acquire a target injection speed and a target injection volume for contrast;
a first processing module, configured to determine a first length of a spring and an initial injection speed corresponding to the first length based on the target injection volume and a sectional area of a filling tube, wherein the first length is a length of the spring when a volume of a chamber between one end of the filling tube and a piston is the target injection volume;
a second processing module, configured to determine, when the target injection speed is greater than the initial injection speed, a second length of the spring based on the target injection speed, the initial injection speed, and the first length; and
a third processing module, configured to precompress the spring from the first length to the second length before filling carbon dioxide into the chamber for storing carbon dioxide formed between the end of the filling tube and the piston, wherein the second length is a length of the spring when a carbon dioxide contrast injection is performed by the filling tube.

7. A contrast injector, **characterized in that** it comprises a filling tube for storing and filling carbon dioxide, wherein a piston and a spring for adjusting a capacity of the filling tube are provided within the filling tube, a chamber for storing carbon dioxide is formed between one end of the filling tube and the piston, and the spring is provided at the other end of the filling tube, wherein the spring pushes the piston to move when it deforms; a baffle is provided within the filling tube at an end of the spring close to the piston, a pull rod for adjusting a position of the baffle is fixed to the baffle, and the pull rod extends out of the filling tube from an end of the filling tube away from an injection port; and a driving member is provided at an end of the filling tube away from the piston, and an end of the spring away from the injection port is fixedly connected to a driving end of the driving member, wherein prior to contrast injection, the contrast injector controls the baffle and the driving member by the method for controlling a carbon dioxide contrast injector according to claim 5, so that the spring is compressed to a second length.

8. The contrast injector according to claim 7, **characterized in that** the driving member is an adjustment rod for manual adjustment, one end of the adjustment rod is fixedly connected to the end of the spring away from the injection port, and the other end of the adjustment rod extends out the filling tube from an end of the filling tube away from the injection port; alternatively, the driving member is a motor, wherein a driving end of the motor is fixedly connected to the end of the spring away from the injection port.

9. An electronic device, comprising a memory, a processor, and a computer program stored in the memory and executable on the processor, **characterized in that** the processor, when executing the program, implements the method for controlling a carbon dioxide contrast injector according to any one of claims 1 to 5.

10. A non-transitory computer-readable storage medium, storing a computer program thereon, **characterized in that** the computer program, when executed by a processor, implements the method for controlling a carbon dioxide contrast injector according to any one of claims 1 to 5.

Acquiring a target injection speed and a target injection volume for contrast ⌐110

Determining a first length of the spring and an initial injection speed corresponding to the first length based on the target injection volume and a sectional area of the filling tube ∽120

Determining, when the target injection speed is greater than the initial injection speed, a second length of the spring, based on the target injection speed, the initial injection speed, and the first length ∽130

Pre-compressing the spring from the first length to the second length before filling carbon dioxide into the chamber for storing carbon dioxide formed between the end of the filling tube and the piston ∽140

Figure 1

| Determining a storage position of a carbon dioxide gas within the filling tube based on the target injection volume and the sectional area of the filling tube | 121 |

↓

| Determining the first length of the spring based on the storage position of the carbon dioxide | 122 |

↓

| Obtaining a target pressure difference generated by the spring on the carbon dioxide in the filling tube based on the first length of the spring, an elastic coefficient of the spring, and the sectional area of the filling tube | 123 |

↓

| Obtaining a gas flow rate of the carbon dioxide based on the target pressure difference and a density of carbon dioxide, and obtaining the initial injection speed based on the gas flow rate of the carbon dioxide | 124 |

Figure 2

Figure 3

Figure 4

Figure 5

610

630

Processor

Memory

Communications bus

640

620

Communications
interface

Figure 6

## INTERNATIONAL SEARCH REPORT

| International application No. |
|---|
| **PCT/CN2025/099703** |

| A. | CLASSIFICATION OF SUBJECT MATTER |
|---|---|

A61M5/00(2006.01)i;  G16H20/17(2018.01)i

According to International Patent Classification (IPC) or to both national classification and IPC

| B. | FIELDS SEARCHED |
|---|---|

Minimum documentation searched (classification system followed by classification symbols)

IPC:A61M5/-,G16H20/-

Documentation searched other than minimum documentation to the extent that such documents are included in the fields searched

Electronic data base consulted during the international search (name of data base and, where practicable, search terms used)

CNKI, CNTXT, USTXT, DWPI, ENTXTC: 北京阿迈特医疗器械有限公司, 刘奕玮, 刘青, 赵庆洪, 冯汉卿, 孙浩, 宫晓菲, 吴文, 林之祺, 二氧化碳, 注射器, 弹簧, 注射量, 注射速度, 截面积, 长度, 距离, 弹性, 系数, 形变, injector, spring, volume, speed, area, length, distance, elastic+, deformation, coefficient

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 118384360 A (BEIJING ADVANCED MEDICAL TECHNOLOGIES, LTD.) 26 July 2024 (2024-07-26) <br> description, paragraphs 29-98, figures 1-6, and claims 1-10 | 1-10 |
| Y | CN 114748737 A (BEIJING ADVANCED MEDICAL TECHNOLOGIES, LTD.) 15 July 2022 (2022-07-15) <br> description, paragraphs 29-48, and figures 1-5 | 1-3, 6, 9-10 |
| Y | CN 118177841 A (BEIJING ADVANCED MEDICAL TECHNOLOGIES, LTD.) 14 June 2024 (2024-06-14) <br> claims 1-10 | 1-3, 6, 9-10 |
| Y | CN 114173847 A (CAREFUSION 303, INC.) 11 March 2022 (2022-03-11) <br> description, paragraphs 69-72, and figure 10 | 1-3, 6, 9-10 |
| A | CN 109200395 A (LU WEN et al.) 15 January 2019 (2019-01-15) <br> entire document | 1-10 |
| A | CN 115955951 A (AESCULA TECH, INC.) 11 April 2023 (2023-04-11) <br> entire document | 1-10 |

☑ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&" document member of the same patent family

| Date of the actual completion of the international search | Date of mailing of the international search report |
|---|---|
| **11 September 2025** | **16 September 2025** |

| Name and mailing address of the ISA/CN | Authorized officer |
|---|---|
| **China National Intellectual Property Administration (ISA/ CN)** <br> **China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| International application No. |
| --- |
| **PCT/CN2025/099703** |

### C.    DOCUMENTS CONSIDERED TO BE RELEVANT

| Category* | Citation of document, with indication, where appropriate, of the relevant passages | Relevant to claim No. |
| --- | --- | --- |
| A | CN 206081229 U (DING PING et al.) 12 April 2017 (2017-04-12)<br>entire document | 1-10 |
| A | CN 211410492 U (BOSHILI BIOTECHNOLOGY DEVELOPMENT (YANCHENG) CO., LTD.) 04 September 2020 (2020-09-04)<br>entire document | 1-10 |
| A | US 10058372 B1 (SHADDUCK, John H.) 28 August 2018 (2018-08-28)<br>entire document | 1-10 |

Form PCT/ISA/210 (second sheet) (July 2022)

## INTERNATIONAL SEARCH REPORT
### Information on patent family members

| International application No. |
| --- |
| **PCT/CN2025/099703** |

| Patent document cited in search report | | | Publication date (day/month/year) | Patent family member(s) | | | Publication date (day/month/year) |
| --- | --- | --- | --- | --- | --- | --- | --- |
| CN | 118384360 | A | 26 July 2024 | None | | | |
| CN | 114748737 | A | 15 July 2022 | None | | | |
| CN | 118177841 | A | 14 June 2024 | None | | | |
| CN | 114173847 | A | 11 March 2022 | AU | 2020268886 | A1 | 02 December 2021 |
| | | | | AU | 2020268886 | B2 | 05 June 2025 |
| | | | | CA | 3139064 | A1 | 12 November 2020 |
| | | | | WO | 2020227104 | A1 | 12 November 2020 |
| | | | | US | 2025170336 | A1 | 29 May 2025 |
| | | | | JP | 2022530993 | A | 05 July 2022 |
| | | | | JP | 7500612 | B2 | 17 June 2024 |
| | | | | US | 2023233771 | A1 | 27 July 2023 |
| | | | | US | 12220562 | B2 | 11 February 2025 |
| | | | | EP | 3962555 | A1 | 09 March 2022 |
| | | | | US | 2020345941 | A1 | 05 November 2020 |
| | | | | US | 11642466 | B2 | 09 May 2023 |
| | | | | MX | 2021013446 | A | 10 December 2021 |
| CN | 109200395 | A | 15 January 2019 | None | | | |
| CN | 115955951 | A | 11 April 2023 | TW | 202216231 | A | 01 May 2022 |
| | | | | WO | 2021252841 | A1 | 16 December 2021 |
| | | | | CA | 3182467 | A1 | 16 December 2021 |
| | | | | US | 2023233374 | A1 | 27 July 2023 |
| | | | | EP | 4164561 | A1 | 19 April 2023 |
| CN | 206081229 | U | 12 April 2017 | None | | | |
| CN | 211410492 | U | 04 September 2020 | None | | | |
| US | 10058372 | B1 | 28 August 2018 | US | 2019053842 | A1 | 21 February 2019 |
| | | | | WO | 2019036527 | A1 | 21 February 2019 |

Form PCT/ISA/210 (patent family annex) (July 2022)

**EP 4 691 514 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202410828438